(19) Europäisches Patentamt
European Patent Office
Office européen des brevets

(11) Numéro de publication : **0 132 168**
**B1**

(12) # FASCICULE DE BREVET EUROPÉEN

(45) Date de publication du fascicule du brevet : **16.06.87**

(21) Numéro de dépôt : **84401201.3**

(22) Date de dépôt : **13.06.84**

(51) Int. Cl.⁴ : **C 07 C121/48**, C 07 C121/75, C 07 F 9/40, A 01 N 53/00, A 23 K 1/16, A 61 K 31/275// C07D213/64, C07D233/74

(54) **Esters d'acides cyclopropane carboxyliques comportant un groupement cyano, procédé et intermédiaires de préparation, leur application comme pesticides et les compositions les renfermant.**

(30) Priorité : **14.06.83 FR 8309810**

(43) Date de publication de la demande : **23.01.85 Bulletin 85/04**

(45) Mention de la délivrance du brevet : **16.06.87 Bulletin 87/25**

(84) Etats contractants désignés : **AT BE CH DE FR GB IT LI LU NL SE**

(56) Documents cités :
**EP-A- 0 003 420**
**EP-A- 0 008 867**
**GB-A- 2 099 810**
**Le dossier contient des informations techniques présentées postérieurement au dépôt de la demande et ne figurant pas dans le présent fascicule.**

(73) Titulaire : **ROUSSEL-UCLAF**
**35, boulevard des Invalides**
***F-75007 Paris (FR)***

(72) Inventeur : **Tessier, Jean**
**30, rue Jean Moulin**
**F-94300-Vincennes (FR)**
Inventeur : **Demoute, Jean-Pierre**
**249bis, rue de Rosny**
**F-93100-Montreuil-sous-Bois (FR)**
Inventeur : **Cadiergue, Joseph**
**6, rue Jules Princet**
**F-93600-Aulnay-sous-Bois (FR)**

(74) Mandataire : **Tonnellier, Marie-José et al**
***Département des Brevets* ROUSSEL UCLAF *B.P. no* 9**
**F-93230 Romainville (FR)**

**EP 0 132 168 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

L'invention concerne des esters d'acides cyclopropane carboxyliques comportant un groupement cyané sur la chaîne latérale du cyclopropane, leur procédé et leurs intermédiaires de préparation, leur application comme pesticides et les compositions les renfermant.

On connaissait déjà des esters dérivés de l'acide 2-cyano éthényl 2,2-diméthyl cyclopropane carboxylique (cf. les brevets E.P.A. 000 342, E.P.A. 008 867 et CB-A 2 099 810) présentant des propriétés pesticides intéressantes.

On vient de découvrir de nouveaux esters dérivés de l'acide 2-cyano éthényl 2,2-diméthyl cyclopropane carboxylique portant sur le carbone en 2 du radical éthényl une fonction oxygénée doués de propriétés pesticides tout à fait inattendues.

L'invention a pour objet, sous toutes leurs formes isomères, les composés de formule générale (I) :

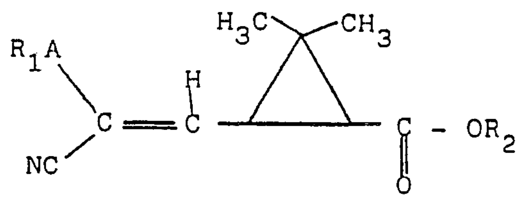

(I)

dans laquelle A représente un atome d'oxygène un groupement sulfoxyde ou un groupement sulfone et ou bien $R_1$ représente un radical alcoyle linéaire, ramifié ou cyclisé, saturé ou insaturé, renfermant jusqu'à 18 atomes de carbone et dont la chaîne peut être interrompue par un ou plusieurs hétéroatomes, ou substituée par un ou plusieurs atomes d'halogène,

ou bien $R_1$ représente un radical aryle ou aralcoyle renfermant de 6 à 18 atomes de carbone et $R_2$ représente un atome d'hydrogène, un radical alcoyle linéaire, ramifié ou cyclisé, saturé ou insaturé, renfermant jusqu'à 12 atomes de carbone ou un radical choisi dans le groupe des radicaux suivants

A. Les radicaux

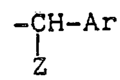

dans lesquels Z représente un atome d'hydrogène, un radical éthynyle ou cyano et Ar représente un radical phényl, pentafluorophényl, ou

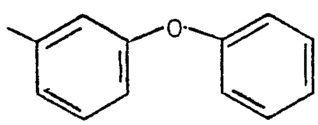

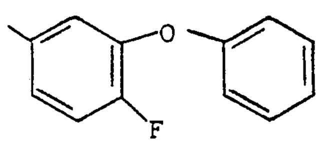

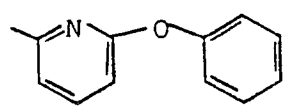

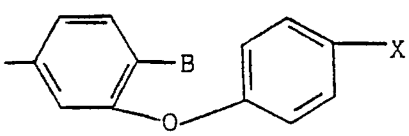

dans lequel B représente un atome d'hydrogène ou de fluor et X représente un atome de fluor, de chlore ou de brome,

B. Le radical

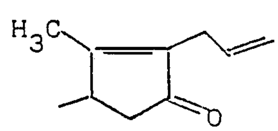

C. Le radical

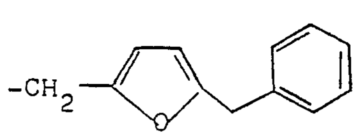

D. et les radicaux

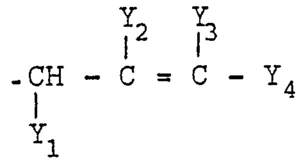

formule dans laquelle $Y_1$ représente un atome d'hydrogène, un atome de fluor, de chlore ou de brome, un groupement —C≡N ou un groupement —C≡CH, $Y_2$, $Y_3$, $Y_4$ identiques ou différents, représentent un atome d'hydrogène, un atome de fluor, de chlore ou de brome, un radical alcoyle linéaire, ramifié ou cyclique, renfermant de 1 à 8 atomes de carbone, éventuellement substitué par un ou plusieurs groupements fonctionnels, identiques ou différents, un radical alcényle comportant de 2 à 8 atomes de carbone ou un radical alcycnyle comportant de 2 à 8 atomes de carbone, les radicaux $Y_2$, $Y_3$ et $Y_4$ peuvent former les cycles entre eux deux à deux.

Lorsque $R_2$ représente un radical alcoyl linéaire, ramifié, ou cyclisé, saturé ou insaturé, il s'agit de préférence du radical méthyle, éthyle, propyle, linéaire ou ramifié, butyle, linéaire ou ramifié, pentyle, linéaire ou ramifié, hexyle, linéaire ou ramifié, cyclopropyle, cyclobutyle, cyclopentyle, cyclohexyle, 1-propényle, 1-butynyle, 1,3-butadiényle, 1-pentényle, 1-cyclobutynyle, 1-cyclopentadiényle, 1-cyclohexényle.

Dans les composés de formule (I), $R_1$ peut représenter notamment des radicaux méthyle, éthyle, propyle linéaire ou ramifié, butyle linéaire ou ramifié, pentyle linéaire ou ramifié, hexyle linéaire ou ramifié, cyclopropyle, cyclobutyle, cyclopentyle, cyclohexyle, 1-propényle, 1-butényle, 1,3-butadiényle, 1-pentényle, 1-cyclobutényle, 1-cyclopentényle, 1-cyclohexényle, un groupement $—CH_2—O—CH_3$, un groupement $—CH_2—CH_2—O—CH_3$, un groupement $—CH_2—NH—CH_3$, un groupement $—CH_2—CH_2—NH—CH_3$, un groupement $—CH_2—S—CH_3$, un groupement $—CH_2—CH_2—S—CH_3$, un groupement

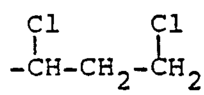

un groupement

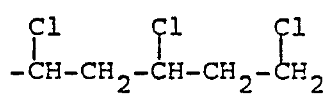

un groupement

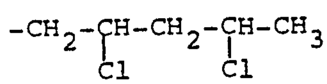

un groupement

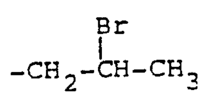

un groupement

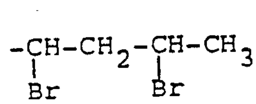

un groupement

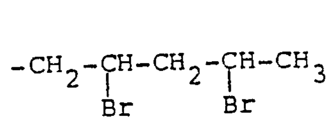

$R_1$ peut représenter également un radical phényle, tolyle, xylyle, benzyle, un groupement

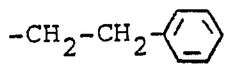

un groupement

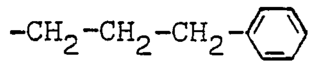

Lorsque $Y_2$, $Y_3$ ou $Y_4$ représente un radical alcoyle linéaire, ramifié ou cyclique éventuellement substitué par un ou plusieurs groupements fonctionnels, il s'agit de préférence d'un radical méthyle, éthyle, isopropyle, n-butyle, isobutyle ou tert-butyle, substitué éventuellement par un ou plusieurs groupements fonctionnels et l'on entend par groupement fonctionnel un atome d'halogène, un groupement OH ou SH, un groupement OR′ ou SR′ dans lesquels R′ représente un radical alcoyle renfermant de 1 à 8 atomes de carbone, un groupement $—NO_2$,

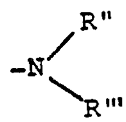

dans lequel R″ et R‴, identiques ou différents, représentent un atome d'hydrogène ou un radical alcoyle renfermant de 1 à 8 atomes de carbone, un groupement —C≡N, $SO_3H$ ou $PO_4H_2$ ou un groupement $—COalc_1$, $SO_2alc_2$ ou $SO_3alc_3$ dans lesquels $alc_1$, $alc_2$ et $alc_3$ représentent des radicaux alcoyles renfermant de 1 à 18 atomes de carbone.

Lorsque $Y_2$, $Y_3$ ou $Y_4$ représente un radical alcényle, il s'agit de préférence du radical vinyle, allyle ou 2-butényle.

Lorsque $Y_2$, $Y_3$ ou $Y_4$ représente un radical alcynyle, il s'agit de préférence du radical éthynyle, propargyle ou 2-butynyle.

Les composés de formule (I) peuvent exister sous de nombreuses formes stéréoisomères, ils possèdent, en effet, deux carbones asymétriques en 1 et en 3 du cyclopropane ; ils présentent aussi une possibilité d'isomérie E/Z au niveau de la double liaison ; ils peuvent présenter également une ou plusieurs possibilités d'isomérie dans la partie alcoolique.

L'invention a notamment pour objet les composés de formule (I) dans lesquels A représente un atome d'oxygène.

L'invention a plus spécialement pour objet les composés de formule ($I_1$) :

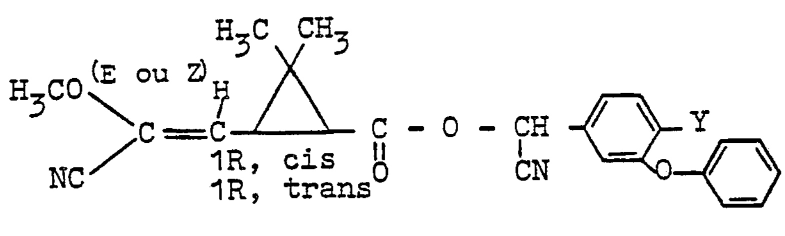

($I_1$)

dans laquelle Y représente un atome d'hydrogène ou un atome de fluor et notamment, parmi ceux-ci, les composés de formule ($I_2$) :

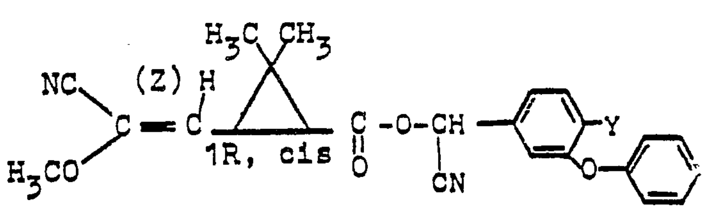

($I_2$)

dans laquelle Y représente un atome d'hydrogène ou un atome de fluor.

L'invention a tout particulièrement pour objet les composés dont la préparation est donnée plus loin dans la partie expérimentale et notamment, parmi ceux-ci, les composés de formule générale (I) dont les noms suivent :

le 1R, cis 3-(ΔE 2-méthoxy 2-cyano éthényl) 2,2-diméthyl cyclopropane carboxylate de (S) cyano 1-(3-phénoxyphényl) méthyle ;

le 1R, cis 3-(ΔZ 2-méthoxy 2-cyano éthényl) 2,2-diméthyl cyclopropane carboxylate de (S) cyano 1-(3-phénoxyphényl) méthyle ;

le 1R, cis 3-(ΔZ 2-méthoxy 2-cyano éthényl) 2,2-diméthyl cyclopropane carboxylate de (S) cyano 1-(3-phénoxy 4-fluorophényl) méthyle ;

le 1R, cis 3-(ΔZ 2-méthoxy 2-cyano éthényl) 2,2-diméthyl cyclopropane carboxylate de (2,3,4,5,6-pentafluorophényl) méthyle ;

le 1R, trans 3-(ΔE 2-méthoxy 2-cyano éthényl) 2,2-diméthyl cyclopropane carboxylate de (S) cyano 1-(3-phénoxyphényl) méthyle.

L'invention a également pour objet un procédé de préparation des composés de formule générale (I), caractérisé en ce que l'on fait réagir, au sein d'un solvant organique, un α-cyano α-$AR_1$ méthyl phosphonate de dialcoyle de formule :

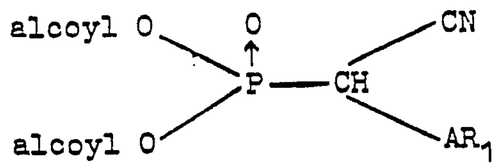

dans laquelle A et $R_1$ conservent les significations précitées et alcoyl représente un radical alcoyle comportant de 1 à 8 atomes de carbone ou bien les deux radicaux alcoyles représentent ensemble une chaîne aliphatique éventuellement substituée par des radicaux alcoyle, avec l'acide 2,2-diméthyl 3-formyl cyclopropane 1-carboxylique de formule :

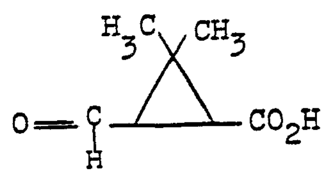

sous forme cis ou trans, en présence d'un agent basique, isole le mélange d'acides bruts obtenus de formule :

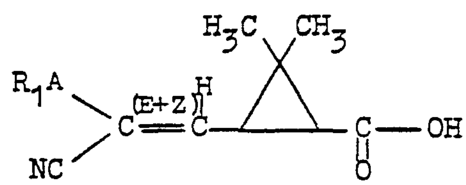

dans laquelle A et $R_1$ conservent les significations précitées, dont on sépare, si désiré, les isomères ΔE et ΔZ puis, le cas échéant, fait réagir les acides ΔE et ΔZ ou leurs mélanges, tels quels ou sous forme de dérivés fonctionnels, avec les alcools $R_2OH$ ou avec un dérivé fonctionnel de ces alcools, pour obtenir les esters de formule (I) sous forme ΔE ou ΔZ ou de leurs mélanges que si désiré l'on sépare, pour obtenir les esters ΔE et ΔZ.

Comme agent basique, on peut utiliser, par exemple, un terbutylate alcalin comme le terbutylate de potassium ou encore un organolithien comme le butyllithium.

Dans des modes de réalisation préférés de l'invention,

pour obtenir un produit de formule (I) de configuration cis, on fait réagir un α-cyano α-$AR_1$ méthyl phosphonate de dialcoyle avec le (1R, 5S) 6,6-diméthyl 4(R)-hydroxy 3-oxabicyclo [3.1.0.] hexan-2-one de formule :

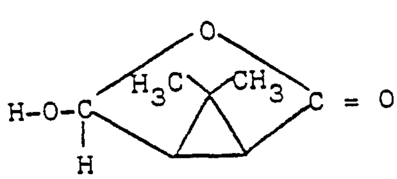

en présence de terbutylate de potassium, acidifie le mélange réactionnel, isole le mélange d'acides bruts obtenu, de formule :

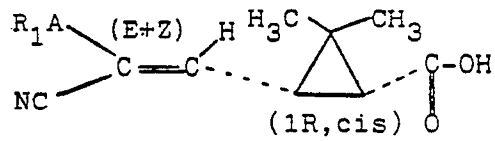

dont on sépare, si désiré, les isomères ΔE et ΔZ, puis fait réagir, le cas échéant, ces isomères séparés ou leur mélange, tels quels ou sous forme de dérivés fonctionnels, avec l'alcool $R_2OH$ ou avec un dérivé fonctionnel de cet alcool ;

pour obtenir un produit de formule (I) de configuration trans, on fait réagir un α-cyano α-$AR_1$ méthyl phosphonate de dialcoyle avec le butyllithium, puis fait réagir le produit obtenu avec le 1R, trans 2,2-diméthyl 3-formyl cyclopropane carboxylate de terbutyle de formule :

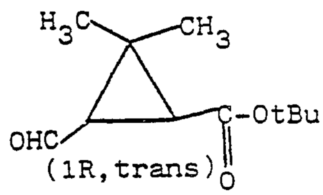

isole le mélange d'acide brut obtenu, de formule

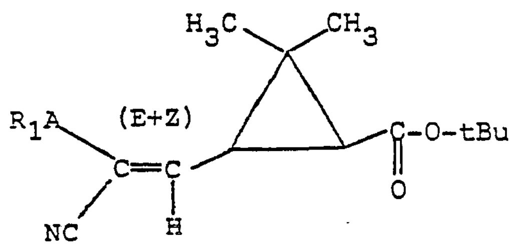

dont on sépare, si désiré, les isomères ΔE etΔZ, puis si désiré, clive le reste terbutyle soit par l'acide trifluoroacétique pour les isomères ΔZ soit par l'acide paratoluène sulfonique pour les isomères ΔE, et ensuite, le cas échéant, soumet les acides ΔE et ΔZ ainsi obtenus ou un dérivé fonctionnel de ces acides, à l'action de l'alcool $R_2OH$ ou d'un dérivé fonctionnel de cet alcool, pour obtenir le composé de formule (I) correspondant.

La séparation des mélanges d'acides bruts, pour obtenir les dérivés « ΔE » et « ΔZ » est réalisée par chromatographie.

Les phosphonates utilisés comme produits de départ sont connus d'une façon générale et peuvent être préparés selon le procédé décrit par S. Watt dans J. Org. Chem. 41, 28 46 (1976).

Certains phosphonates sont nouveaux et sont en eux-mêmes un objet de la présente invention à savoir :

le 2-(cyano 2-propyloxy méthyl) 2-oxo 4,5-diméthyl 1,3,2-dioxa phospholane ;

le 2-(cyano éthoxyméthyl) 2-oxo 4,5-diméthyl 1,3,2-dioxaphospholane.

L'invention a plus particulièrement pour objet ces phosphonates particuliers comme produits intermédiaires nécessaires à la mise en œuvre du procédé de l'invention.

Les composés de formule (I) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc aussi pour objet l'application des composés de formule (I) tels que définis précédemment, à la lutte contre les parasites des végétaux, les parasites des locaux et les parasites des animaux à sang chaud.

Les produits de formule (I) peuvent être utilisés notamment pour lutter contre les insectes dans le domaine agricole, pour lutter par exemple contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) sont de plus photostables et ne sont pas toxiques pour les mammifères.

L'ensemble de ces propriétés fait que les produits de formule (I) correspondent parfaitement aux exigences de l'industrie agrochimique moderne : ils permettent de protéger les récoltes tout en préservant l'environnement.

Les produits de formule (I) peuvent aussi être utilisés pour lutter contre des acariens et les nématodes

parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique. Ils peuvent aussi être utilisés contre les poux et les helminthes.

L'invention a donc également pour objet les compositions destinées à la lutte contre les parasites des végétaux, les parasites des locaux et les parasites des animaux à sang chaud, caractérisées en ce qu'elles renferment comme principe actif au moins un des produits de formule générale (I).

L'invention a notamment pour objet les compositions insecticides renfermant comme principe actif au moins l'un des produits de formule générale (I).

Parmi les compositions notamment insecticides préférées de l'invention, on peut citer tout spécialement les compositions renfermant les composés décrits dans les exemples et plus particulièrement les composés de formule générale (I) dont les noms suivent :

le 1R, cis 3-(ΔE 2-méthoxy 2-cyanoéthényl) 2,2-diméthyl cyclopropane carboxylate de (S) cyano 1-(3-phénoxyphényl) méthyle ;

le 1R, cis 3-(ΔZ 2-méthoxy 2-cyanoéthényl) 2,2-diméthyl cyclopropane carboxylate de (S) cyano 1-(3-phénoxyphényl) méthyle ;

le 1R, cis 3-(ΔZ 2-méthoxy 2-cyanoéthényl) 2,2-diméthyl cyclopropane carboxylate de (S) cyano 1-(3-phénoxy 4-fluorophényl) méthyle ;

le 1R, cis 3-(ΔZ 2-méthoxy 2-cyanoéthényl) 2,2-diméthyl cyclopropane carboxylate de (2,3,4,5,6-pentafluorophényl) méthyle ;

le 1R, trans 3-(ΔE 2-méthoxy 2-cyanoéthényl) 2,2-diméthyl cyclopropane carboxylate de (S) cyano 1-(3-phénoxyphényl) méthyle.

Ces compositions sont préparées selon les procédés usuels de l'industrie agrochimique. Elles peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en générale un véhicule et/ou un agent tensioactif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin combustible, ou encore d'un substrat fibreux incombustible. Dans ce dernier cas, le fumigant obtenu après incorporation de la matière active est placé sur un appareil chauffant.

Dans le cas où l'on utilise un serpentin insecticide, le support inerte peut être, par exemple, composé de marc de pyrèthre, poudre de Tabu (ou poudre de feuilles de Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut être, par exemple, de 0,03 à 25 % en poids.

Les compositions selon l'invention pour un usage dans les locaux peuvent aussi être obtenues en préparant une huile pulvérisable à base de principe actif, cette huile imbibant la mèche d'une lampe qui est alors soumise à la combustion.

La concentration du principe actif incorporé à l'huile est, de préférence, de 0,03 à 25 % en poids.

L'invention a aussi pour objet les compositions acaricides renfermant comme principe actif au moins l'un des produits de formule générale (I), ainsi que les compositions nématicides renfermant comme principe actif au moins l'un des produits de formule générale (I).

Les compositions acaricides et nématicides peuvent se présenter notamment sous forme de poudre, granulés, suspensions, émulsions, solutions.

Comme pour les compositions insecticides, les compositions acaricides et nématicides peuvent être additionnées éventuellement d'un ou plusieurs autres agents pesticides.

Pour l'usage acaricide, on utilise de préférence des poudres mouillables, pour pulvérisation foliaire, contenant de 1 à 80 % de principe actif ou des liquides pour pulvérisation foliaire contenant de 1 à 500 g/l de principe actif. On peut également employer des poudres pour poudrage foliaire contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention, sont utilisés, de préférence, à des doses comprises entre 1 et 100 g de matière active à l'hectare.

L'invention a également pour objet les compositions acaricides renfermant comme principe actif l'un au moins des composés de formule (I), caractérisées en ce qu'elles sont utilisées dans la lutte contre les parasites des animaux à sang chaud, notamment contre les tiques et les gales.

Les compositions de l'invention peuvent être utilisées sous forme de Spray de bains ou encore de badigeonnage selon la méthode « pour on ».

Lorsqu'on utilise la méthode « pour on », on utilise de préférence des solutions renfermant de 0,5 à 4 g de principe actif pour 100 $cm^3$ de solution.

Lorsqu'il s'agit de lutter contre les acariens parasites des animaux, on peut incorporer les produits de l'invention dans des compositions alimentaires en association avec un mélange nutritif adapté à l'alimentation animale. Le mélange nutritiel peut varier selon l'espèce animale ; il peut renfermer des céréales, des sucres et des grains, des tourteaux de soja, d'arachide et de tournesol, des farines d'origine animale, par exemple, des farines de poissons, des acides aminés de synthèse, des sels minéraux, des vitamines et des antioxydants.

L'invention a donc ainsi également pour objet les compositions destinées à l'alimentation animale renfermant comme principe actif au moins l'un des produits de formule générale (I).

L'invention a également pour objet les associations douées d'activité insecticide, acaricide ou nématicide, caractérisées en ce qu'elles contiennent comme matière active, d'une part un au moins des composés de formule générale (I) et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane 1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane 1-carboxyliques, par les esters d'alcools α-cyano 3-phénoxy benzyliques d'acides 2,2-diméthyl 3-(2,2-dibromovinyl) cyclopropane 1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane 1-carboxyliques dans lesquels « halo » représente un atome de fluor, de chlore ou de brome, étant entendu que les copules acides et alcools des esters pyréthrinoïdes ci-dessus peuvent exister sous toutes leurs formes stéréoisomères possibles.

L'invention a enfin pour objet les compositions ou associations définies précédemment, caractérisées en ce qu'elles renferment en outre un synergiste des pyréthrinoïdes.

Comme synergistes classiques utilisés en pareil cas, on peut citer le 1-(2,5,8-trioxadodécyl) 2-propyl 4,5-méthylène dioxy benzène (ou butoxyde de pipéronyle) ou le N-(2-éthylheptyl) bicyclo [2,2-1]-5-heptène 2,3-dicarboximide ou le pipéronyl-bis-2-(2'n-butoxyéthoxy) éthyl acétal (ou tropital).

Les exemples suivants illustrent l'invention sans la limiter.

## Exemple 1

Acide 1R, cis 3-(ΔZ 2-méthoxy 2-cyano éthényl) 2,2-diméthyl cyclopropane carboxylique et acide 1R, cis 3-(ΔE 2-méthoxy 2-cyano éthényl) 2,2-diméthyl cyclopropane carboxylique.

Dans 140 $cm^3$ de tétrahydrofuranne on introduit 10,35 g d'α-cyano α-méthoxy méthyl phosphonate de diéthyle, 7,1 g de (1R, 5S) 6,6-diméthyl 4(R)-hydroxy 3-oxa bicyclo [3-1-0]-hexan 2-one, ajoute à — 60 °C, en 30 minutes environ, 11,2 g de terbutylate de potassium dans 75 $cm^3$ de tétrahydrofuranne, agite pendant 6 heures à — 60 °C, verse le mélange réactionnel dans l'eau glacée, acidifie à pH 5 par addition d'une solution aqueuse 2N d'acide chlorhydrique, extrait à l'éther éthylique, concentre à sec par distillation sous pression réduite, chromatographie le résidu sur silice en éluant par un mélange d'hexane et d'acétate d'éthyle (4/6) et obtient 738 mg d'acide 1R, cis 3-(ΔZ 2-méthoxy 2-cyano éthényl) 2,2-diméthyl cyclopropane carboxylique (F = 74 °C puis 82 °C), 2,88 g d'acide 1R, cis 3-(ΔE 2-méthoxy 2-cyano éthényl) 2,2-diméthyl cyclopropane carboxylique (F = 82 °C) et 3,74 g d'un mélange d'isomères « ΔE » et « ΔZ ».

Isomère « ΔZ ».

Spectre de RMN (deutérochloroforme)

pics à 1,27-1,28 ppm attribués aux hydrogènes des méthyles géminés

pics à 1,83-1,97 ppm et 2,2-2,34 ; 2,37-2,51 ppm attribués aux hydrogènes en positions 1 et 3 du cyclopropyle

pic à 3,8 ppm attribué aux hydrogènes du méthyle du méthoxy

pics à 5,9-6.07 ppm attribués à l'hydrogène éthylénique.

Spectre ultra-violet (éthanol)

maximum à 239-240 nm ε = 18 400

Isomère « ΔE ».

Spectre de RMN (deutérochloroforme)

pic à 1,3 attribué aux hydrogènes des méthyles géminés
pics à 1,82-1,96 ppm et 2,05-2,21-2,37 ppm attribués aux hydrogènes en positions 1 et 3 du cyclopropyle
pic à 3,68 ppm attribué aux hydrogènes du méthyle du méthoxy
pics à 5,9-6,09 ppm attribués à l'hydrogène éthylénique
Spectre ultra-violet (éthanol)
Maximum à 240-241 nm ε = 12 500.

Exemple 2

1R, trans 3-(ΔE 2-méthoxy 2-cyanoéthényl) 2,2-diméthyl cyclopropane carboxylate de terbutyle et 1R, trans 3-(ΔZ 2-méthoxy 2-cyano éthényl) 2,2-diméthyl cyclopropane carboxylate de terbutyle.

Stade A : préparation de l'anion.

Dans 100 $cm^3$ de tétrahydrofuranne, on dissout 10,36 g d'α-cyano α-méthoxy méthyle phosphonate de diéthyle, introduit à —60 °C, en 30 minutes environ, 33 $cm^3$ d'une solution à 15 % de butyl lithium dans l'hexane (titrant 1,6 mole/l), agite pendant 10 minutes à —60 °C et obtient la solution A utilisée pour la condensation suivante.

Stade B : condensation.

Dans la solution A obtenue précédemment, on introduit à —60 °C, en 40 minutes environ, une solution de 9,9 g de 2,2-diméthyl 3-formyl cyclopropane-1-carboxylate de terbutyle dans 50 $cm^3$ de tétrahydrofuranne, agite pendant 5 heures à —60 °C, verse le mélange réactionnel dans l'eau glacée, extrait à l'éther, concentre à sec par distillation sous pression réduite, chromatographie le résidu sur gel de silice en éluant avec un mélange d'hexane et d'acétate d'éthyle (95/5) et obtient 2,98 g de 1R, trans 3-(ΔZ 2-méthoxy 2-cyano éthényl) 2,2-diméthyl cyclopropane carboxylate de terbutyle (F = 57 °C), et 7,8 g de 1R, trans 3-(ΔE 2-méthoxy 2-cyano éthényl) 2,2-diméthyl cyclopropane carboxylate de terbutyle sous forme d'huile.
Isomère « ΔZ »
Spectre de RMN (deutérochloroforme)
pics à 1,18-1,27 ppm attribués aux hydrogènes des méthyles géminés
pic à 1,48 ppm attribué aux hydrogènes des méthyles du terbutyle
pic à 3,8 ppm attribué aux hydrogènes du méthyle du méthoxy
pics à 5,2-5,37 ppm attribués à l'hydrogène éthylénique
Spectre ultra-violet (éthanol)
maximum à 239 nm, $E_1^1$ = 686 soit ε = 17 300
Isomère « ΔE »
Spectre de RMN (deutérochloroforme)
pics à 1,17-1,3 ppm attribués aux hydrogènes des méthyles géminés
pic à 1,48 ppm attribué aux hydrogènes des méthyles du terbutyle
pic à 3,67 ppm attribué aux hydrogènes du méthyle du méthoxy
pics à 5,28-5,43 ppm attribués à l'hydrogène éthylénique
Spectre ultra-violet (éthanol)
maximum à 240 nm, $E_1^1$ = 470 soit ε = 11 800

Exemple 3

Acide 1R, trans 3-(ΔZ 2-méthoxy 2-cyano éthényl) 2,2-diméthyl cyclopropane carboxylique.

Dans 60 $cm^3$ de chlorure de méthylène on dissout 3 g de 1R, trans 3-(ΔZ 2-méthoxy 2-cyano éthényl) 2,2-diméthyl cyclopropane carboxylate de terbutyle, introduit lentement à 0 °C, 14,6 $cm^3$ d'acide trifluoroacétique, agite pendant 3 heures à 0 °C, concentre à sec par distillation sous pression réduite, ajoute du cyclohexane, concentre à sec par distillation sous pression réduite, chromatographie le résidu sur silice en éluant par un mélange d'hexane et d'acétate d'éthyle (4/6) et obtient 2,05 g d'acide 1R, trans 3-(ΔZ 2-méthoxy 2-cyano éthényl) 2,2-diméthyl cyclopropane carboxylique (F = 110 °C).

Exemple 4

Acide 1R, trans 3-(ΔE 2-méthoxy 2-cyano éthényl) 2,2-diméthyl cyclopropane carboxylique

Dans 50 $cm^3$ de toluène on dissout 5 g de 1R, trans 3-(ΔE 2-méthoxy 2-cyano éthényl) 2,2-diméthyl cyclopropane carboxylate de terbutyle, ajoute 0,5 g d'acide para toluène sulfonique, porte le mélange réactionnel au reflux, l'y maintient pendant 45 minutes, refroidit, concentre à sec par distillation sous

pression réduite, ajoute au résidu de l'eau et de l'éther, agite, sépare par décantation la phase organique, concentre à sec par distillation sous pression réduite et obtient 3,8 g d'acide 1R, trans 3-(ΔE 2-méthoxy 2-cyano éthényl) 2,2-diméthyl cyclopropane carboxylique (F = 125 °C).

## Exemple 5

Acide 1R, cis 3-(ΔZ 2-éthoxy 2-cyano éthényl) 2,2-diméthyl cyclopropane carboxylique et acide 1R, cis 3-(ΔE 2-éthoxy 2-cyano éthényl) 2,2-diméthyl cyclopropane carboxylique.

Dans 14 $cm^3$ de tétrahydrofuranne, on introduit 1,93 g de bromure de lithium puis à — 30 °C, 1,4 $cm^3$ de diisopropylamine, 1,42 g de (1R, 5S) 6,6-diméthyl 4(R)-hydroxy 3-oxa bicyclo [3-1-0]-hexan 2-one en solution dans 10 $cm^3$ de tétrahydrofuranne, 2,19 g de 2-(cyano éthoxy) méthyl 2-oxo 4,5-diméthyl 1,3,2-dioxaphospholane, agite pendant 30 minutes à — 30 °C, introduit une solution de 2,24 g de terbutylate de potassium dans 15 $cm^3$ de tétrahydrofuranne, agite pendant 2 heures à — 15 °C, verse le mélange réactionnel dans une solution aqueuse 2N d'acide chlorhydrique, extrait à l'éther, lave à l'eau, concentre à sec par distillation sous pression réduite, chromatographie le résidu sur silice en éluant par un mélange d'hexane et d'acétate d'éthyle (4/6), puis par un mélange d'hexane et d'acétate d'éthyle (8/2) à 1 % d'acide acétique et obtient 430 mg d'acide ΔZ recherché et 560 mg d'acide ΔE recherché.

Isomère Z

Spectre de RMN (deutérochloroforme)

pics à 1,29-1,31 ppm des hydrogènes des méthyles géminés
pics à 1,3-1,33-1,45 ppm des hydrogènes du méthyle de l'éthoxy
pics à 1,81-2,5 ppm des hydrogènes en 1 et en 3 du cyclopropane
pics à 3,85-3,98-4,1-4,2 ppm des hydrogènes du méthylène de l'éthoxy
pics à 5,86-6,03 ppm de l'hydrogène éthylénique

Isomère E

Spectre de RMN (deutérochloroforme)

pics à 1,2-1,32-1,35 ppm des hydrogènes du méthyle de l'éthoxy
pic à 1,28 ppm des hydrogènes des méthyles géminés
pics à 1,8-2,33 ppm des hydrogènes en 1 et en 3 du cyclopropane
pics à 3,7-3,8-3,9-4,05 ppm des hydrogènes du méthylène de l'éthoxy
pics à 5,9-6,1 ppm de l'hydrogène éthylénique.

Le 2-(cyano éthoxy) méthyl 2-oxo 4,5-diméthyl 1,3,2-dioxaphospholane utilisé comme produit de départ a été préparé comme suit :

On mélange 26,11 g de α-bromo α-éthoxy acétonitrile et 24,22 g de 2-méthoxy 4,5-diméthyl 1,3,2-dioxaphospholane, chauffe lentement à 100 °C et maintient pendant une heure, refroidit, rectifie sous pression réduite et obtient 27,5 g de 2-(cyano éthoxy)méthyl 2-oxo 4,5-diméthyl 1,3,2-dioxaphospholane (eb = 151 °C sous 0,4 mm de mercure).

## Exemple 6

Mélange d'acide 1R, cis 3-[ΔZ 2-isopropyloxy) 2-cyano 1-éthényl] 2,2-diméthyl cyclopropane carboxylique et d'acide 1R, cis 3-[ΔE 2-(2-isopropyloxy)2-cyano 1-éthényl] 2,2-diméthyl cyclopropane carboxylique.

Dans 330 $cm^3$ de tétrahydrofuranne, on introduit 33,1 g de bromure de lithium, ajoute à — 30 °C, 14 $cm^3$ de diisopropylamine, agite pendant 30 minutes à — 30 °C, introduit à — 30 °C, en 20 minutes 23,76 g de 2-(cyano 2-isopropyloxy méthyl) 2-oxo 4,5-diméthyl 1,3,2-dioxaphospholane et 14,24 g de (1R, 5S) 6,6-diméthyl 4(R)-hydroxy 3-oxa bicyclo [3-1-0] hexan-2-one, agite pendant 30 minutes à — 30 °C, introduit en 30 minutes environ, à — 30 °C, une solution de 22,55 g de terbutylate de potassium dans 220 $cm^3$ de tétrahydrofuranne, agite pendant 3 heures à — 20 °C, verse le mélange réactionnel dans une solution aqueuse 2N d'acide chlorhydrique, extrait à l'éther éthylique, lave à l'eau, concentre à sec par distillation sous pression réduite, chromatographie le résidu sur silice en éluant dans un mélange d'hexane et d'acétate d'éthyle (4/6) et obtient 14,49 g d'un mélange d'acide (1R, cis) 3-[ΔZ 2-(2-isopropyloxy 2-cyano 1-éthényl)] 2,2-diméthyl cyclopropane carboxylique et d'acide 1R, cis 3-[ΔE 2-(2-isopropyloxy 2-cyano 1-éthényl)] 2,2-diméthyl cyclopropane carboxylique.

Spectre de RMN (deutérochloroforme)

pics à 1,22-1,35 ppm des hydrogènes des méthyles géminés
pics à 1,8-2,5 ppm des hydrogènes en 1 et en 3 du cyclopropane
pics à 4,0-4,7 ppm de l'hydrogène du > CH de l'isopropyle
pics à 5,97-6,13 et de 6,10-6,26 ppm de l'hydrogène éthylénique.

Les données de ce spectre correspondent à un rapport des deux isomères de 55/45.

Préparation du 2-(cyano 2-isopropyloxy méthyl) 2-oxo 4,5-diméthyl 1,3,2-dioxaphospholane.

On mélange 24,47 g de α-bromo α-isopropyloxy acétonitrile, 20,63 g de 2-méthoxy 4,5-diméthyl

1,3,2-dioxaphospholane, porte le mélange réactionnel à 100 °C, maintient cette température pendant une heure, refroidit, rectifie sous pression réduite et obtient 23,76 g de 2-(cyano 2-propyloxy)méthyl 2-oxo 4,5-diméthyl 1,3,2-dioxaphospholane (eb. 130 °C sous 0,3 mm de mercure).

Exemple 7

Acide 1R cis 3-[ΔE + ΔZ 2-terbutyloxy 2-cyano éthényl] 2,2-diméthyl cyclopropane carboxylique.

En opérant comme à l'exemple 6 à partir du 1R cis 6,6-diméthyl 4(R)-hydroxy 3-oxa bicyclo [3,1,0] hexan-2-one et d'α-cyano α-terbutoxy méthyl phosphonate de diéthyle on a obtenu le produit recherché.

Exemple 8

1R, cis 3-(ΔZ 2-méthoxy 2-cyano éthényl) 2,2-diméthyl cyclopropane carboxylate de 3-phénoxy phényl méthyle.

On mélange 690 mg d'acide 1R, cis 3-(ΔZ 2-méthoxy 2-cyano éthényl) 2,2-diméthyl cyclopropane carboxylique, 7 $cm^3$ de chlorure de méthylène, 700 mg d'alcool 3-phénoxy benzylique, 30 mg de 4-diméthyl amino pyridine, ajoute à 0 °C une solution de 750 mg de dicyclohexylcarbodiimide dans 2 $cm^3$ de chlorure de méthylène, agite pendant 3 heures à 20 °C, filtre, concentre à sec par distillation sous pression réduite, chromatographie le résidu sur silice en éluant par un mélange d'hexane et d'acétate d'éthyle (8/2) et obtient 1,2 g d'ester recherché.

$[\alpha]_D = -20\ °C \pm 1°$ (c = 1,2 %, chloroforme).

Exemples 9 à 42

En opérant comme à l'exemple 8, à partir des acides de formule :

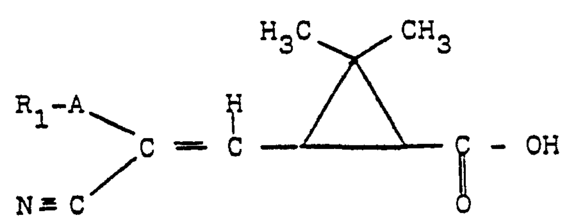

et des alcools de formule $R_2OH$, on a obtenu les esters de formule générale I suivants :

Exemple 9

1R, cis 3-(ΔE 2-(méthoxy 2-cyanoéthényl) 2,2-diméthyl cyclopropane carboxylate de (S) cyano 3-phénoxy-phénylméthyle).

Exemple 10

1R, cis 3-(ΔZ 2-(méthoxy 2-cyanoéthényl) 2,2-diméthyl cyclopropane carboxylate de (S) cyano 3-phénoxy-phénylméthyle).

Exemple 11

1R, cis 3-(ΔE 2-(méthoxy 2-cyanoéthényl) 2,2-diméthyl cyclopropane carboxylate de (S) cyano 3-phénoxy 4-fluorophénylméthyle).

Exemple 12

1R, cis 3-(ΔZ 2-(méthoxy 2-cyanoéthényl) 2,2-diméthyl cyclopropane carboxylate de (S) cyano 3-phénoxy 4-fluorophénylméthyle).

Exemple 13

1R, cis 3-(ΔZ 2-méthoxy 2-cyanoéthényl) 2,2-diméthyl cyclopropane carboxylate de (R, S) cyano (6-phénoxy 2-pyridyl) méthyle.

Exemple 14

1R, cis 3-(ΔZ 2-méthoxy 2-cyanoéthényl) 2,2-diméthyl cyclopropane carboxylate de pentafluorobenzyle.

Exemple 15

1R, cis 3-(ΔZ 2-méthoxy 2-cyanoéthényl) 2,2-diméthyl cyclopropane carboxylate de S-alléthrolone.

Exemple 16

1R, cis 3-(ΔE 2-méthoxy 2-cyanoéthényl) 2,2-diméthyl cyclopropane carboxylate de S-alléthrolone.

Exemple 17

1R, cis (ΔZ 2-méthoxy 2-cyanoéthényl) 2,2-diméthyl cyclopropane carboxylate de 1-(2-propynyl) 2,4-dioxo imidazolidin-3-yl méthyle.

Exemple 18

1R, cis 3-(ΔZ 2-méthoxy 2-cyanoéthényl) 2,2-diméthyl cyclopropane carboxylate de (6-phénoxy 2-pyridyl) méthyle.

Exemple 19

1R, cis 3-(ΔE 2-méthoxy 2-cyanoéthényl) 2,2-diméthyl cyclopropane carboxylate de (6-phénoxy 2-pyridyl) méthyle.

Exemple 20

1R, cis 3-(ΔE 2-méthoxy 2-cyanoéthényl) 2,2-diméthyl cyclopropane carboxylate de (R) éthynyl (3-phénoxyphényl) méthyle.

Exemple 21

1R, cis 3-(ΔZ 2-méthoxy 2-cyanoéthényl) 2,2-diméthyl cyclopropane carboxylate de (R) éthynyl 3-(phénoxy-phényl) méthyle.

Exemple 22

1R, cis 3-(ΔE 2-méthoxy 2-cyanoéthényl) 2,2-diméthyl cyclopropane carboxylate de (R) 1-(3-phénoxyphényl) éthyle.

Exemple 23

1R, cis 3-(ΔZ 2-méthoxy 2-cyanoéthényl) 2,2-diméthyl cyclopropane carboxylate de (R) 1-(3-phénoxyphényl) éthyle.

Exemple 24

1R, trans 3-(ΔE 2-méthoxy 2-cyanéthényl) 2,2-diméthyl cyclopropane carboxylate de (S) cyano-3-phénoxy-phényl méthyle.

Exemple 25

1R, trans 3-(ΔZ 2-méthoxy 2-cyanoéthényl) 2,2-diméthyl cyclopropane carboxylate de (S) cyano 3-phénoxyphényl méthyle.

Exemple 26

1R, trans 3-(ΔE 2-méthoxy 2-cyanoéthényl) 2,2-diméthyl cyclopropane carboxylate de (S) cyano 3-phénoxy 4-fluorophénylméthyle.

Exemple 27

1R, trans 3-(ΔZ 2-méthoxy 2-cyanoéthényl) 2,2-diméthyl cyclopropane carboxylate de (S) cyano 3-phénoxy

4-fluorophénylméthyle.

Exemple 28

1R, cis 3-(ΔE 2-éthoxy 2-cyanoéthényl) 2,2-diméthyl cylopropane carboxylate de (S) cyano 3-phénoxyphényl méthyle.

Exemple 29

1R, cis 3-(ΔZ 2-éthoxy 2-cyanoéthényl) 2,2-diméthyl cyclopropane carboxylate de (S) cyano 3-phénoxyphényl méthyle.

Exemple 30

1R, cis 3-(ΔZ 2-éthoxy 2-cyanoéthényl) 2,2-diméthyl cyclopropane carboxylate de (S) cyano 3-phénoxy 4-fluorophényl méthyle.

Exemple 31

1R, cis 3-(ΔZ 2-éthoxy 2-cyanoéthényl) 2,2-diméthyl cyclopropane carboxylate de (R, S) cyano (6-phénoxy 2-pyridyl) méthyle.

Exemple 32

1R, cis 3-(ΔE 2-isopropoxy 2-cyanoéthényl) 2,2-diméthyl cyclopropane carboxylate de (S) cyano (3-phénoxyphényl) méthyle.

Exemple 33

1R, cis 3-(ΔZ 2-isopropoxy 2-cyanoéthényl) 2,2-diméthyl cyclopropane carboxylate de (S) cyano (3-phénoxyphényl) méthyle.

Exemple 34

1R, cis 3-(ΔE 2-isopropoxy 2-cyanoéthényl) 2,2-diméthyl cyclopropane carboxylate de (S) cyano (3-phénoxy 4-fluorophényl) méthyle.

Exemple 35

1R, cis 3-(ΔZ 2-isopropoxy 2-cyanoéthényl) 2,2-diméthyl cyclopropane carboxylate de (S) cyano (3-phénoxy-4-fluorophényl) méthyle.

Exemple 36

1R, cis 3-(ΔE 2-isopropoxy 2-cyanoéthényl) 2,2-diméthyl cyclopropane carboxylate de (RS) cyano (6-phénoxy 2-pyridyl) méthyle.

Exemple 37

1R, cis 3-(ΔZ 2-isopropoxy 2-cyanoéthényl) 2,2-diméthyl cyclopropane carboxylate de (RS) cyano (6-phénoxy 2-pyridyl) méthyle.

Exemple 38

1R, cis 3-(ΔE 2-terbutoxy 2-cyanoéthényl) 2,2-diméthyl cyclopropane carboxylate de (S) cyano (3-phénoxyphényl) méthyle.

Exemple 39

1R, cis 3-(ΔZ 2-terbutoxy 2-cyanoéthényl) 2,2-diméthyl cyclopropane carboxylate de (S) cyano (3-phénoxyphényl) méthyle.

Exemple 40

1R, cis 3-(ΔE 2-terbutoxy 2-cyanoéthényl) 2,2-diméthyl cyclopropane carboxylate de (S) cyano (3-phénoxy

4-fluorophényl) méthyle.

Exemple 41

1R, cis 3-(ΔZ 2-terbutoxy 2-cyanoéthényl) 2,2-diméthyl cyclopropane carboxylate de (S) cyano (3-phénoxy 4-fluorophényl) méthyle.

Exemple 42

1R, cis 3-(ΔE+Z 2-terbutoxy 2-cyanoéthényl) 2,2-diméthyl cyclopropane carboxylate de (RS) cyano (6-phénoxy 2-pyridyl) méthyle.

(Voir tableaux pages 15, 16, 17)

| Exemple | A | $R_1$ | Δ | Configuration | $R_2$ | Point de fusion | $(\alpha)_D$ | Concentration dans $CHCl_3$ |
|---|---|---|---|---|---|---|---|---|
| 9 | O | $CH_3$ | E | 1R, cis | 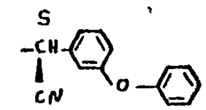 | F = 76° C | +45° | 1% |
| 10 | " | " | Z | " | " | – | +27° | 0,5% |
| 11 | " | " | E | " | 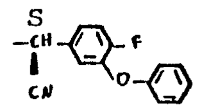 | – | +46,5° | 0,7% |
| 12 | " | " | Z | " | " | – | +36° | 0,8% |
| 13 | " | " | Z | " | 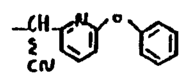 | – | 0 ° | 1% |
| 14 | " | " | Z | " | pentafluorobenzyle | – | | |
| 15 | " | " | Z | " | S-alléthrolone | – | +14,5° | 0,6% |
| 16 | " | " | E | " | " | – | +65° | 0,5% |
| 17 | " | " | Z | " | 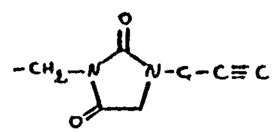 | – | | |
| 18 | " | " | Z | " | 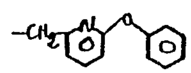 | – | -2,5° | 0,6% |
| 19 | " | " | E | " | " | – | +51° | 0,6% |

| Exemple | A | $R_1$ | Δ | Configu-ration | $R_2$ | Point de fusion | $(\alpha)_D$ | Concentration dans $CHCl_3$ |
|---|---|---|---|---|---|---|---|---|
| 20 | O | $CH_3$ | E | 1R, cis | 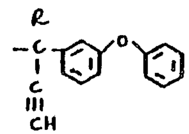 | — | +45° | 0,75% |
| 21 | " | " | Z | " | " | — | +8,5° | 1 % |
| 22 | " | " | E | " | 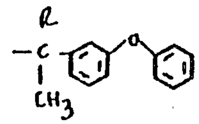 | — | +125,5° | 0,65% |
| 23 | " | " | Z | " | " | — | +121,5° | 0,8 % |
| 24 | " | " | E | 1R, trans | 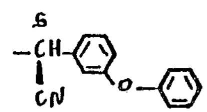 | — | -5,5° | 1,4 % |
| 25 | " | " | Z | " | " | — | 0° | |
| 26 | " | " | E | " | 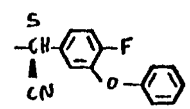 | F = 107° | -6,5° | 1,7 % |
| 27 | " | " | Z | " | " | — | +2° | 0,4 % |
| 28 | " | $C_2H_5$ | E | 1R, cis | 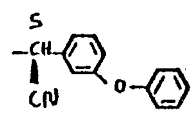 | — | +41° | 0,7 % |
| 29 | " | " | Z | " | " | — | +38° | 0,7 % |

| Exemple | A | $R_1$ | Δ | Configu-ration | $R_2$ | Point de fusion | $(\alpha)_D$ | Concentration dans $CHCl_3$ |
|---|---|---|---|---|---|---|---|---|
| 30 | O | $-CH_2-CH_3$ | Z | 1R, cis | 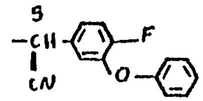 | – | +41,5° | 0,6% |
| 31 | " | " | Z | " | 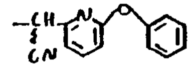 | – | + 5° | 0,5% |
| 32 | " | $CH(CH_3)_2$ | E | " | 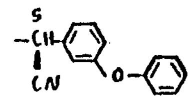 | – | +51° | 1 % |
| 33 | " | " | Z | " | " | F = 92° | +50,5° | 0,5% |
| 34 | " | " | E | " | 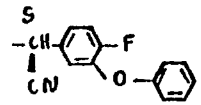 | – | +56,5° | 0,6% |
| 35 | " | " | Z | " | " | – | +43,5° | 0,7% |
| 36 | " | " | E | " | 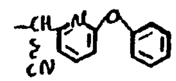 | – | +49,5° | 0,5% |
| 37 | " | " | Z | " | " | – | +26,5° | 0,5% |
| 38 | " | $C(CH_3)_3$ | E | " | 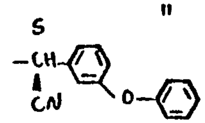 | F = 72° | +64° | 0,5% |
| 39 | " | " | Z | " | " | – | +40° | 0,5% |
| 40 | " | " | E | " | 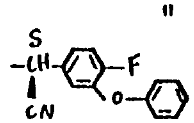 | – | +61° | 0,5% |
| 41 | " | " | Z | " | " | – | +39° | 0,5% |
| 42 | " | " | E+Z | " | 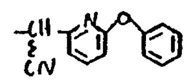 | – | +26,5° | 0,6% |

Pour préparer les isomères géométriques des exemples 9 et 42, si l'on est parti de l'acide E ou Z pur, on obtient l'ester E ou Z correspondant, si l'on est parti de l'acide E + Z, on obtient un mélange d'esters d'acide E + Z que l'on sépare par des procédés physiques comme la chromatographie.

Exemple 43

préparation d'un concentré soluble

On effectue un mélange homogène de :

| | |
|---|---|
| produit de l'exemple 9 | 0,25 g |
| butoxyde de pipéronyle | 1 g |
| tween 80 | 0,25 g |
| topanol A | 0,1 g |
| eau | 98,4 g |

Exemple 44

préparation d'un concentré émulsifiable

On mélange intimement :

| | |
|---|---|
| produit de l'exemple 14 | 0,015 g |
| butoxyde de pipéronyle | 0,5 g |
| topanol A | 0,1 g |
| xylène | 99,385 g |

Exemple 45

préparation d'un concentré émulsifiable

On effectue un mélange homogène de :

| | |
|---|---|
| produit de l'exemple 12 | 1,5 g |
| tween 80 | 20 g |
| topanol A | 0,1 g |
| xylène | 78,4 g |

Exemple 46

préparation d'une composition fumigène

| | |
|---|---|
| produit de l'exemple 10 | 0,25 g |
| poudre de tabu | 25 g |
| poudre de feuille de cèdre | 40 g |
| poudre de bois de pin | 33,75 g |
| vert brillant | 0,5 g |
| p-nitrophénol | 0,5 g |

Etude de l'activité insecticide des composés de l'invention

A) Etude de l'effet d'abattage sur mouche domestique.

Les insectes tests sont des mouches domestiques femelles âgées de 4 jours. On opère par pulvérisation directe à la concentration de 0,25 g/l en chambre de Kearns et March en utilisant comme solvant un mélange d'acétone (5 %) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Les résultats expérimentaux obtenus sont résumés dans le tableau suivant :

| Produit de l'exemple | KT 50 |
|---|---|
| 9 | 2,12 |
| 10 | 2,29 |
| 24 | 1,85 |
| 12 | 2,45 |
| 14 | 1,91 |

B) Etude de l'effet létal des composés de l'invention sur divers insectes.

a) Etude de l'effet létal sur blatte.

Les tests sont effectués par contact sur film sur verre, par dépôt à la pipette, de solutions acétoniques de différentes concentrations sur fond de boîte de Petri en verre dont les bords ont été préalablement talqués afin d'éviter la fuite des insectes. On détermine la concentration létale 50 (CL 50).

Les résultats expérimentaux obtenus sont résumés dans le tableau suivant :

| Produit de l'exemple | CL 50 en $mg/m^2$ |
|---|---|
| 9 | 3,4 |
| 10 | 0,50 |
| 24 | 4,0 |
| 12 | 0,38 |
| 14 | 1,91 |

b) Etude de l'effet létal sur larves de Spodoptera Littoralis.

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24 °C et 65 % d'humidité relative. Après traitement les individus sont placés sur un milieu nutritif artificiel (milieu de Poitout).

On effectue le contrôle des mortalités 48 heures après traitement.

Les résultats expérimentaux obtenus sont résumés dans le tableau suivant :

| Produit de l'exemple | DL 50 en ng par insecte |
|---|---|
| 9 | 10,0 |
| 10 | 1,03 |
| 24 | 19,7 |
| 12 | 2,79 |
| 14 | 24,1 |

c) Etude de l'effet létal sur Aphis Cracivora.

On utilise des adultes après 7 jours et l'on emploie 10 Aphis par concentration utilisée. On utilise une méthode de contact-ingestion. On effectue le traitement au pistolet de Fisher d'une feuille de fève que l'on dépose dans une boîte de Petri en matière plastique sur une rondelle de papier humidifiée. Le traitement est effectué à l'aide de 2 ml de solution acétonique de produit à tester (1 ml par face de feuille).

L'infestation par insecte est effectuée après séchage de la feuille. On maintient les insectes en contact avec la feuille pendant une heure. On place les insectes sur des feuilles non traitées et contrôle la mortalité au bout de 24 heures.

Les résultats expérimentaux obtenus sont résumés dans le tableau suivant :

| Produit de l'exemple | DL 50 en ng par insecte |
|---|---|
| 10 | 0,69 |
| 12 | 2,62 |
| 14 | 5,15 |

**Revendications**

1. Sous toutes leurs formes isomères, les composés de formule générale (I) :

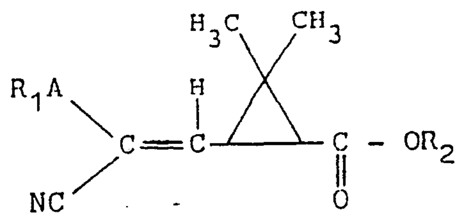

(I)

dans laquelle A représente un atome d'oxygène un groupement sulfoxyde ou un groupement sulfone et ou bien $R_1$ représente un radical alcoyle linéaire, ramifié ou cyclisé, saturé ou insaturé, renfermant jusqu'à 18 atomes de carbone et dont la chaîne peut être interrompue par un ou plusieurs hétéroatomes, ou substituée par un ou plusieurs atomes d'halogène,
ou bien $R_1$ représente un radical aryle ou aralcoyle renfermant de 6 à 18 atomes de carbone
et $R_2$ représente un atome d'hydrogène, un radical alcoyle linéaire, ramifié ou cyclisé, saturé ou insaturé, renfermant jusqu'à 12 atomes de carbone ou un radical choisi dans le groupe des radicaux suivants :

A. Les radicaux

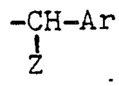

dans lesquels Z représente un atome d'hydrogène, un radical éthynyle ou cyano et Ar représente un radical phényl, pentafluorophényl ou

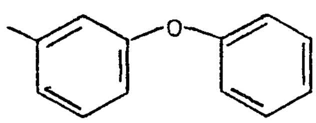

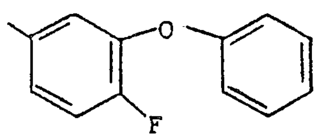

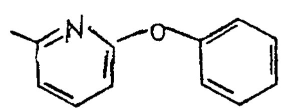

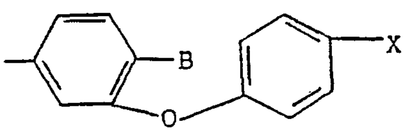

dans lequel B représente un atome d'hydrogène ou de fluor et X représente un atome de fluor, de chlore ou de brome,

B. Le radical :

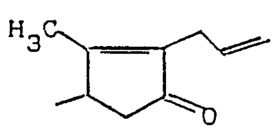

C. Le radical :

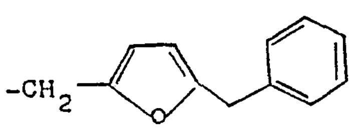

D. et les radicaux :

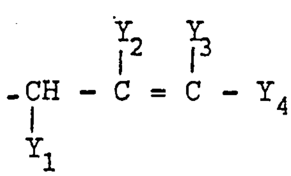

formule dans laquelle $Y_1$ représente un atome d'hydrogène, un atome de fluor, de chlore ou de brome, un groupe —C≡N ou un groupement —C≡CH, $Y_2$, $Y_3$, $Y_4$ identiques ou différents, représentent un atome d'hydrogène, un atome de fluor, de chlore ou de brome, un radical alcoyle linéaire, ramifié ou cyclique, renfermant de 1 à 8 atomes de carbone, éventuellement substitué par un ou plusieurs groupements fonctionnels, identiques ou différents, un radical alcényle comportant de 2 à 8 atomes de carbone ou un radical alcynyle comportant de 2 à 8 atomes de carbone, les radicaux $Y_2$, $Y_3$ et $Y_4$ peuvent former les cycles entre eux deux à deux.

2. Les composés de formule générale (I), conformes à la revendication 1, dans lesquels A représente un atome d'oxygène.

3. Les composés de formule générale (I) conformes à la revendication 1, répondant à la formule ($I_1$) :

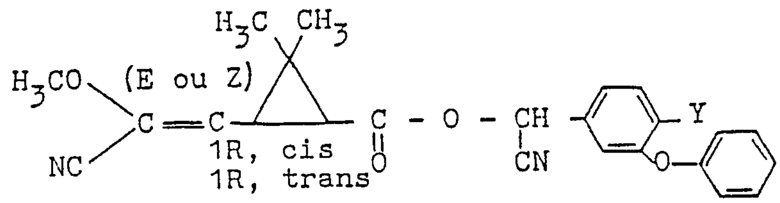

($I_1$)

dans laquelle Y représente un atome d'hydrogène ou un atome de fluor.

4. Les composés de formule générale (I), conformes à la revendication 1, répondant à la formule ($I_2$) :

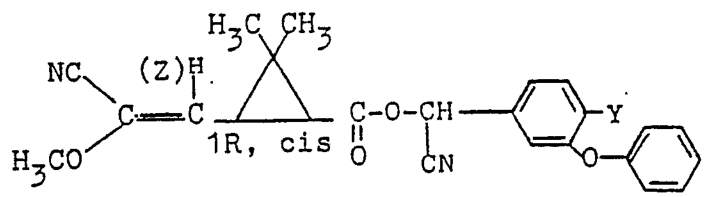

($I_2$)

dans laquelle Y représente un atome d'hydrogène ou un atome de fluor.

5. Les composés de formule générale (I), conformes à la revendication 1, dont les noms suivent :

le 1R, cis 3-(ΔE 2-méthoxy 2-cyano éthényl) 2,2-diméthyl cyclopropane carboxylate de (S) cyano 1-(3-phénoxyphényl) méthyle ;

le 1R, cis 3-(ΔZ 2-méthoxy 2-cyano éthényl) 2,2-diméthyl cyclopropane carboxylate de (S) cyano 1-(3-phénoxyphényl) méthyle ;

le 1R, cis 3-(ΔZ 2-méthoxy 2-cyano éthényl) 2,2-diméthyl cyclopropane carboxylate de (S) cyano 1-(3-phénoxy 4-fluorophényl) méthyle ;

le 1R, cis 3-(ΔZ 2-méthoxy 2-cyano éthényl) 2,2-diméthyl cyclopropane carboxylate de (2,3,4,5,6-pentafluorophényl) méthyle ;

le 1R, trans 3-(ΔE 2-méthoxy 2-cyano éthényl) 2,2-diméthyl cyclopropane carboxylate de (S) cyano 1-(3-phénoxyphényl) méthyle.

6. Procédé de préparation des composés de formule générale (I), caractérisé en ce que l'on fait réagir, au sein d'un solvant organique, un α-cyano α-$AR_1$ méthyl phosphonate de dialcoyle de formule :

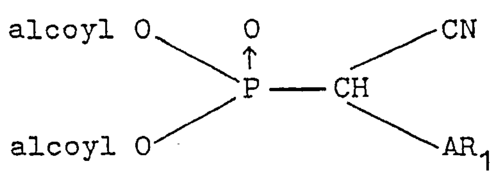

dans laquelle A et $R_1$ conservent les significations de la revendication 1 et alcoyl représente un radical alcoyle comportant de 1 à 8 atomes de carbone ou bien les deux radicaux alcoyles représentent ensemble une chaîne aliphatique éventuellement substituée par des radicaux alcoyle, avec l'acide 2,2-diméthyl 3-formyl cyclopropane 1-carboxylique de formule :

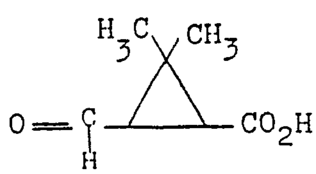

sous forme cis ou trans, en présence d'un agent basique, isole le mélange d'acides bruts obtenu de formule :

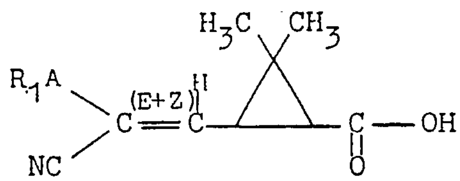

dans laquelle A et $R_1$ conservent les significations précitées, dont on sépare, si désiré, les isomères ΔE et ΔZ puis, le cas échéant, fait réagir les acides ΔE et ΔZ ou leurs mélanges, tels quels ou sous forme de dérivés fonctionnels, avec les alcools $R_2OH$ ou avec un dérivé fonctionnel de ces alcools, pour obtenir les esters de formule (I) sous forme ΔE ou ΔZ ou de leurs mélanges que si désiré l'on sépare, pour obtenir les esters ΔE et ΔZ.

7. Procédé selon la revendication 6, caractérisé en ce que :

pour obtenir un produit de formule (I) de configuration cis, on fait réagir un α-cyano α-$AR_1$ méthyl phosphonate de dialcoyle avec le (1R, 5S) 6,6-diméthyl 4(R)-hydroxy 3-oxabicyclo[3.1.0.]hexan-2-one de formule :

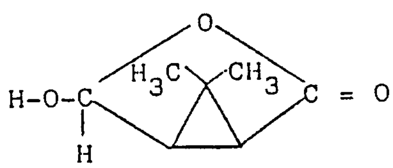

en présence de terbutylate de potassium, acidifie le mélange réactionnel, isole le mélange d'acides bruts obtenus, de formule :

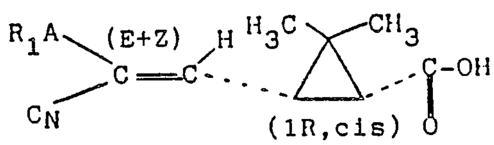

dont on sépare, si désiré, les isomères ΔE et ΔZ, puis fait réagir, le cas échéant, ces isomères séparés ou leur mélange, tels quels ou sous forme de dérivés fonctionnels, avec l'alcool $R_2OH$ ou avec un dérivé fonctionnel de cet alcool ;

pour obtenir un produit de formule (I) de configuration trans, on fait réagir un α-cyano α-$AR_1$ méthyl phosphonate de dialcoyle avec le butyllithium, puis fait réagir le produit obtenu avec le 1R, trans 2,2-diméthyl 3-formyl cyclopropane carboxylate de terbutyle de formule :

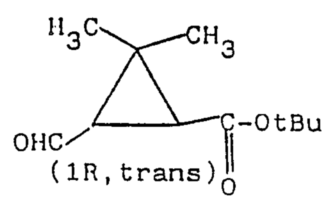

isole le mélange d'acide brut obtenu, de formule

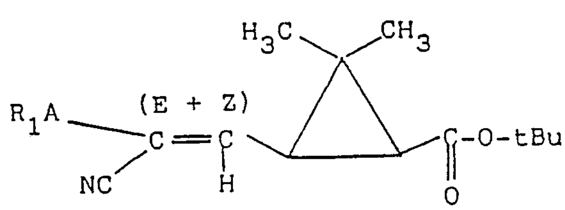

dont on sépare, si désiré, les isomères ΔE et ΔZ, puis si désiré, clive le reste terbutyle soit par l'acide trifluoroacétique pour les isomères ΔZ soit par l'acide paratoluène sulfonique pour les isomères ΔE, et ensuite, le cas échéant, soumet les acides ΔE et ΔZ ainsi obtenus ou un dérivé fonctionnel de ces acides, à

l'action de l'alcool $R_2OH$ ou d'un dérivé fonctionnel de cet alcool, pour obtenir le composé de formule (I) correspondant.

8. Application des composés de formule (I) tels que définis à l'une quelconque des revendications 1 à 5 à la lutte contre les parasites des végétaux et les parasites des locaux.

9. Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 5.

10. Les compositions insecticides renfermant comme principe actif au moins l'un des produits définis à l'une quelconque des revendications 1 à 5.

11. Les compositions acaricides renfermant comme principe actif au moins l'un des produits définis à l'une quelconque des revendications 1 à 5.

12. Les compositions nématicides renfermant comme principe actif au moins l'un des produits définis à l'une quelconque des revendications 1 à 5.

13. Les compositions acaricides destinées à la lutte contre les parasites des animaux à sang chaud, notamment contre les tiques et les gales, caractérisées en ce qu'elles renferment comme principe actif au moins l'un des produits définis à l'une quelconque des revendications 1 à 5.

14. Les compositions destinées à l'alimentation animale renfermant comme principe actif au moins l'un des produits définis à l'une quelconque des revendications 1 à 5.

15. Les compositions selon l'une quelconque des revendications 9 à 14, caractérisées en ce qu'elles renferment en outre un synergiste des pyréthrinoïdes choisi dans le groupe constitué par le butoxyde de pipéronyle ou le tropital.

16. A titre de produits industriels nouveaux,
le 2-(cyano 2-propyloxyméthyl) 2-oxo 4,5-diméthyl 1,3,2-dioxa phospholane,
le 2-(cyano éthoxyméthyl) 2-oxo 4,5-diméthyl 1,3,2-dioxa phospholane.

## Claims

1. In all their isomeric forms, compounds with the general formula (I) :

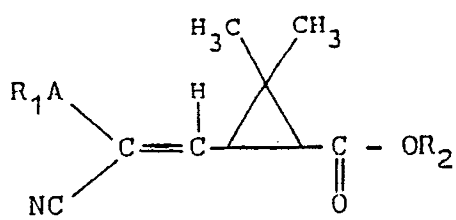

(I)

in which A represents an oxygen atom, a sulphoxide group or a sulphone group and
either $R_1$ represents a linear, branched or cyclized alkyl radical, saturated or unsaturated, containing up to 18 carbon atoms and of which the chain can be interrupted by one or more hetero-atoms, or substituted by one or more halogen atoms,
or $R_1$ represents an aryl or aralkyl radical containing from 6 to 18 carbon atoms
and $R_2$ represents a hydrogen atom, a linear, branched or cyclized alkyl radical, saturated or unsaturated, containing up to 12 carbon atoms or a radical chosen from the following group of radicals :

A. The

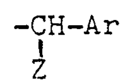

radicals in which Z represents a hydrogen atom, an ethynyl or cyano radical and Ar represents a phenyl, pentafluorophenyl or

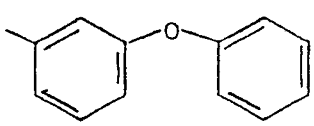 , 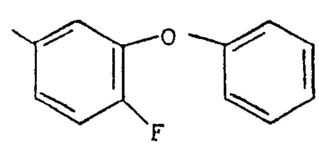 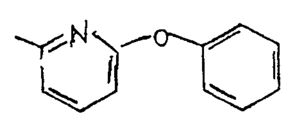

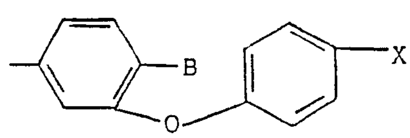

radical,
in which B represents a hydrogen or fluorine atom and X represents a fluorine, chlorine or bromine atom,

B. The radical :

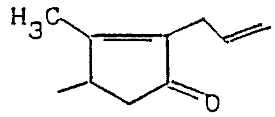

C. The radical :

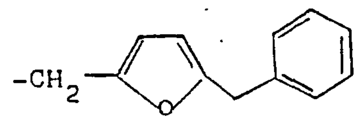

D. and the radicals :

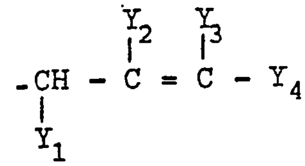

in which formula $Y_1$ represents a hydrogen atom, a fluorine, chlorine or bromine atom, a —C≡N or a —C≡CH group, $Y_2$, $Y_3$, $Y_4$, identical or different, represent a hydrogen atom, a fluorine, chlorine or bromine atom, a linear, branched or cyclic alkyl radical, containing from 1 to 8 carbon atoms, possibly substituted by one or more functional groups, identical or different, an alkenyl radical containing from 2 to 8 carbon atoms or an alkynyl radical containing from 2 to 8 carbon atoms, the radicals $Y_2$, $Y_3$ and $Y_4$ can form rings between them, two by two.

2. Compounds with the general formula (I), conforming to claim 1, in which A represents an oxygen atom.

3. Compounds with the general formula (I), conforming to claim 1, answering to the formula ($I_1$) :

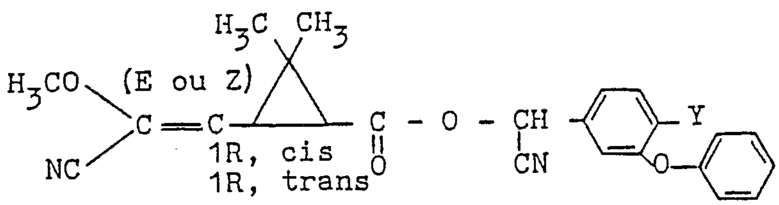

($I_1$)

in which Y represents a hydrogen atom or a fluorine atom.

4. Compounds with the general formula (I), conforming to claim 1, answering to the formula ($I_2$) :

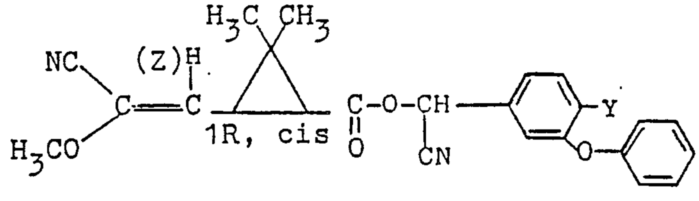

($I_2$)

in which Y represents a hydrogen atom or a fluorine atom.

5. Compounds with the general formula (I), conforming to claim 1, of which the names follow :

1R, cis 3-(ΔE-2-methoxy-2-cyanoethenyl)-2,2-dimethyl cyclopropane carboxylate of (S) cyano-1-(3-phenoxyphenyl) methyl ;

1R, cis 3-(ΔZ-2-methoxy-2-cyanoethenyl)-2,2-dimethyl cyclopropane carboxylate of (S) cyano-1-(3-phenoxyphenyl) methyl ;

1R, cis 3-(ΔZ-2-methoxy-2-cyanoethenyl)-2,2-dimethyl cyclopropane carboxylate of (S) cyano-1-(3-phenoxy-4-fluorophenyl) methyl ;

1R, cis 3-(ΔZ-2-methoxy-2-cyanoethenyl)-2,2-dimethyl cyclopropane carboxylate of (2,3,4,5,6-pentafluorophenyl) methyl ;

1R, trans 3-(ΔE-2-methoxy-2-cyanoethenyl)-2,2-dimethyl cyclopropane carboxylate of (S) cyano-1-(3-phenoxyphenyl) methyl.

6. Preparation process for compounds with the general formula (I), characterized in that a dialkyl α-cyano-α-AR$_1$ methyl phosphonate with the formula :

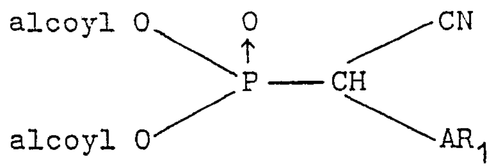

in which A and R$_1$ retain the significances of claim 1 and alkyl represents an alkyl radical containing from 1 to 8 carbon atoms or the two alkyl radicals represent together an aliphatic chain possibly substituted by the alkyl radicals, is made to react, in an organic solvent, with 2,2-dimethyl-3-formyl cyclopropane 1-carboxylic acid with the formula :

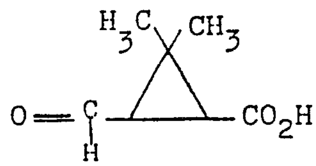

in cis or trans form, in the presence of a basic agent, the mixture of crude acids obtained with the formula :

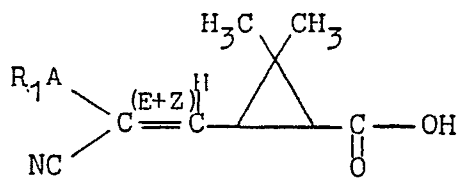

is isolated, in which A and R$_1$ retain the significances already given, of which the ΔE and ΔZ isomers are separated, if desired, then, if desired, the ΔE and ΔZ acids or their mixtures, as they are or in the form of functional derivatives, are made to react with the R$_2$OH alcohols or with a functional derivative of these alcohols, so as to obtain the esters with the formula (I) in the ΔE or ΔZ form or their mixtures which, if desired, are separated, so as to obtain the ΔE nd ΔZ esters.

7. Process according to claim 6, characterized in that :

in order to obtain a product with the formula (I) of cis configuration, a dialkyl α-cyano-α-AR$_1$ methyl phosphonate is made to react with (1R, 5S) 6,6-dimethyl-4(R)-hydroxy-3-oxabicyclo-[3.1.0.]-hexan-2-one with the formula :

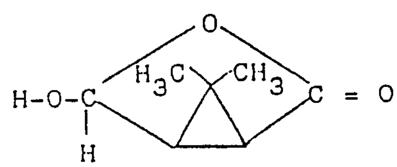

in the presence of potassium tertbutylate, the reactional mixture is acidified, the mixture of crude acids obtained with the formula :

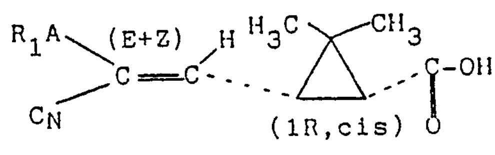

is isolated, from which, if desired, the ΔE and ΔZ isomers are separated, then, if desired, these separated isomers or their mixture, as they are or in the form of functional derivatives, are made to react with the R$_2$OH alcohol or with a functional derivative of this alcohol ;

in order to obtain a product with the formula (I) of trans configuration a dialkyl α-cyano-α-AR$_1$ methyl phosphonate is made to react with butyllithium, then the product obtained is made to react with 1R, trans 2,2--dimethyl-3-formyl cyclopropane carboxylate of tertbutyl with the formula :

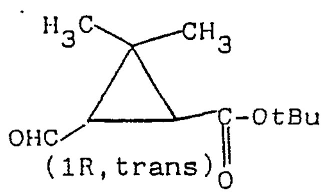

the mixture of crude acid obtained, with the formula :

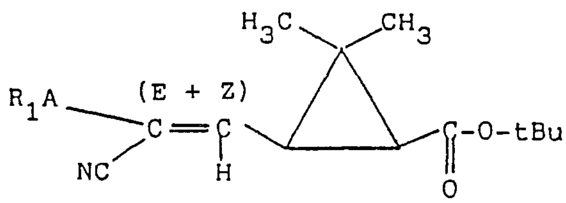

is isolated, from which, if desired, the ΔE and ΔZ isomers are separated, then if desired, the tertbutyl residue is cleaved either by trifluoroacetic acid for the ΔZ isomers or by paratoluene sulphonic acid for the ΔE isomers, and then, if desired, the ΔE and ΔZ acids thus obtained or a functional derivative of these acids, are submitted to the action of the $R_2OH$ alcohol or of a functional derivative of this alcohol, so as to obtain the corresponding compound with the formula (I).

8. Use of the compounds with the formula (I) as defined in any one of the claims 1 to 5 for combatting parasites of vegetation and parasites of premises.

9. Compositions intended for combatting parasites of vegetation, parasites of premises and parasites of warm-blooded animals, characterized in that they contain as active principle at least one of the products defined in any one of the claims 1 to 5.

10. Insecticide compositions containing as active principle at least one of the products defined in any one of the claims 1 to 5.

11. Acaricide compositions containing as active principle at least one of the products defined in any one of the claims 1 to 5.

12. Nematocide compositions containing as active principle at least one of the products defined in any one of the claims 1 to 5.

13. Acaricide compositions intended for combatting parasites of warm-blooded animals, in particular for combatting ticks and mites, characterized in that they contain as active principle at least one of the products defined in any one of the claims 1 to 5.

14. Compositions intended for animal fodder containing as active principle at least one of the products defined in any one of the claims 1 to 5.

15. Compositions according to any one of the claims 9 to 14, characterized in that they contain in addition a pyrethrinoid synergist chosen from the group constituted by piperonyl butoxide or tropital.

16. As new industrial products,
2-(cyano-2-propyloxylmethyl)-2-oxo-4,5-dimethyl-1,3,2-dioxa phospholane,
2-(cyano ethoxymethyl)-2-oxo-4,5-dimethyl-1,3,2-dioxa phospholane.

**Patentansprüche**

1. In sämtlichen ihrer isomeren Formen die Verbindungen der allgemeinen Formel (I)

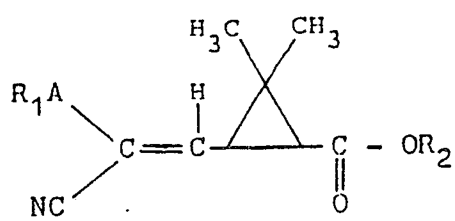

(I)

worin A ein Sauerstoffatom, eine Sulfoxidgruppe oder eine Sulfongruppe bedeutet und
entweder $R_1$ eine gesättigten oder ungesättigten, linearen, verzweigten oder cyclischen Alkylrest mit bis zu 18 Kohlenstoffatomen, dessen Kette durch ein oder mehrere Heteroatome unterbrochen oder durch ein oder mehrere Halogenatome substituiert sein kann, bedeutet,
oder $R_1$ eine Aryl- oder Aralkylrest mit 6 bis 18 Kohlenstoffatomen bedeutet und
$R_2$ ein Wasserstoffatom, einen gesättigten oder ungesättigten, linearen, verzweigten oder cyclischen Alkylrest mit bis zu 12 Kohlenstoffatomen oder einen Rest, ausgewählt unter den folgenden Resten, darstellt,

A. den Resten

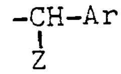

worin Z ein Wasserstoffatom, einen Ethinylrest oder eine Cyanogruppe bedeutet und Ar einen Phenylrest, Pentafluorphenylrest oder

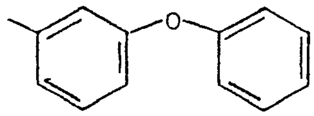

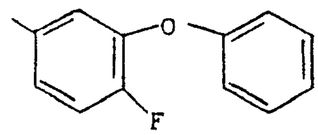

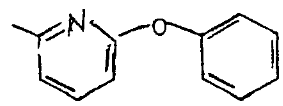

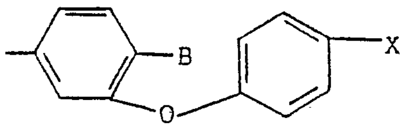

darstellt, worin B ein Wasserstoff- oder Fluoratom bedeutet und X ein Fluor-, Chlor- oder Bromatom bedeutet,

B. dem Rest

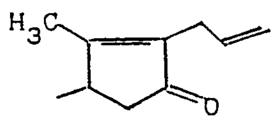

C. dem Rest

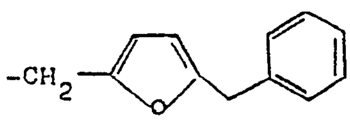

D. und den Resten

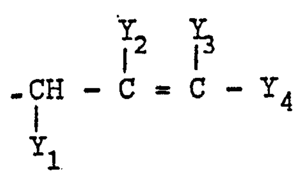

worin $Y_1$ ein Wasserstoffatom, ein Fluor-, Chlor- oder Bromatom, eine Gruppe —C≡N oder eine Gruppe —C≡CH bedeutet, $Y_2$, $Y_3$ und $Y_4$, die gleich oder voneinander verschieden sind, ein Wasserstoffatom, ein Fluor-, Chlor- oder Bromatom, einen linearen, verzweigten oder cyclischen Alkylrest mit 1 bis 8 Kohlenstoffatomen, der gegebenenfalls durch eine oder mehrere gleich oder verschiedene funktionelle Gruppen substituiert sein kann, einen Alkenylrest mit 2 bis 8 Kohlenstoffatomen oder einen Alkinylrest mit 2 bis 8 Kohlenstoffatomen bedeuten, wobei die Reste $Y_2$, $Y_3$ und $Y_4$ paarweise miteinander Ringe bilden können.

2. Verbindungen der allgemeinen Formel (I) gemäß Anspruch 1, worin A ein Sauerstoffatom bedeutet.

3. Verbindungen der allgemeinen Formel (I) gemäß Anspruch 1 der Formel ($I_1$)

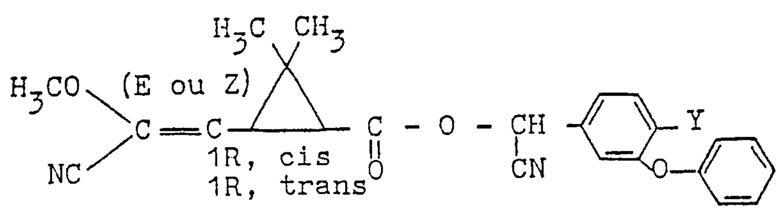

($I_1$)

worin Y ein Wasserstoffatom oder ein Fluoratom bedeutet.

4. Verbindungen der allgemeinen Formel (I) gemäß Anspruch 1 der Formel ($I_2$)

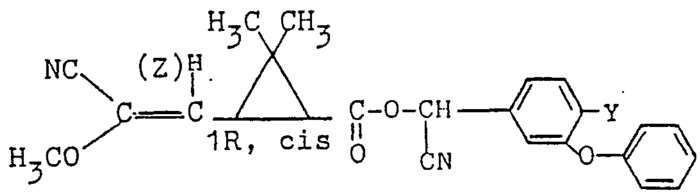

($I_2$)

worin Y ein Wasserstoffatom oder ein Fluoratom bedeutet.

5. Verbindungen der allgemeinen Formel (I) gemäß Anspruch 1 mit den folgenden Bezeichnungen

(S)-Cyano-1-(3-phenoxyphenyl)-methyl-1R, cis-3-(ΔE-2-methoxy-2-cyanoethenyl)-2,2-dimethyl-cyclopropan-carboxylat ;

(S)-Cyano-1-(3-phenoxyphenyl)-methyl-1R, cis-3-(ΔZ-2-methoxy-2-cyanoethenyl)-2,2-dimethyl-cyclopropan-carboxylat ;

(S)-Cyano-1-(3-phenoxy-4-fluorphenyl)-methyl-1R, cis-3-(ΔZ-2-methoxy-2-cyanoethenyl)-2,2-dimethyl-cyclopropan-carboxylat ;

(2,3,4,5,6-Pentafluorphenyl)-methyl-1R, cis-3-(ΔZ-2-methoxy-2-cyanoethenyl)-2,2-dimethyl-cyclopropan-carboxylat ;

(S)-Cyano-1-(3-phenoxyphenyl)-methyl-1R, trans-3-(ΔE-2-methoxy-2-cyanoethenyl)-2,2-dimethyl-cyclopropan-carboxylat.

6. Verfahren zur Herstellung der Verbindungen der allgemeinen Formel (I), dadurch gekennzeichnet, daß man in dem Medium eines organischen Lösungsmittels ein Dialkyl-α-cyano-α-$AR_1$-methylphosphonat der Formel

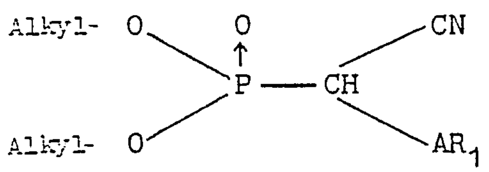

worin A und $R_1$ die in Anspruch 1 angegebenen Bedeutungen besitzen und Alkyl einen Alkylrest mit 1 bis 8 Kohlenstoffatomen darstellt oder zwei Alkylreste gemeinsam eine aliphatische Kette, die gegebenenfalls durch Alkylreste substituiert ist, darstellen, mit der 2,2-Dimethyl-3-formyl-cyclopropan-1-carbonsäure der Formel

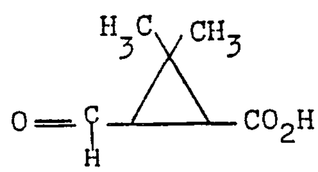

in cis- oder trans-Form in Anwesenheit eines basischen Mittels umsetzt, das erhaltene Gemisch der rohen Säuren der Formel

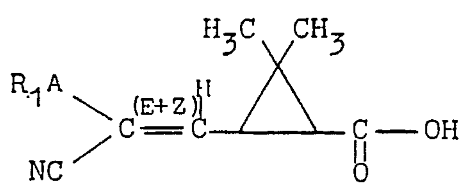

worin A und $R_1$ die vorstehende Bedeutung besitzen, isoliert, hierin gewünschtenfalls die ΔE- und ΔZ-Isomeren trennt, danach gegebenenfalls die ΔE- und ΔZ-Säuren oder deren Gemische als solche oder in Form funktioneller Derivate mit Alkoholen $R_2OH$ oder mit einem funktionellen Derivat dieser Alkohole umsetzt, um die Ester der Formel (I) in der ΔE- oder ΔZ-Form oder deren Gemische zu erhalten, die man gewünschtenfalls trennt, um die ΔE- und ΔZ-Ester zu erhalten.

7. Verfahren gemäß Anspruch 6, dadurch gekennzeichnet, daß man zur Erzielung eines Produkts der Formel (I) mit cis-Konfiguration ein Dialkyl-α-cyano-α-$AR_1$-methylphosphonat mit (1R, 5S)-6,6-Dimethyl-4(R)-hydroxy-3-oxabicyclo[3.1.0]hexan-2-on der Formel

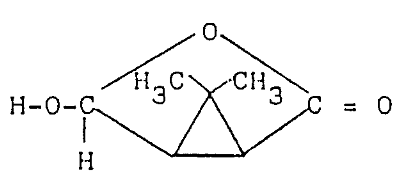

in Anwesenheit von Kalium-tert.-butylat umsetzt, das Reaktionsgemisch ansäuert, das Gemisch der erhaltenen rohen Säuren der Formel

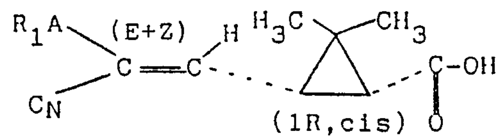

isoliert, in diesem gewünschtenfalls die ΔE- und ΔZ-Isomeren trennt, danach gegebenenfalls diese getrennten Isomeren oder deren Gemisch als solche oder in Form von funktionellen Derivaten mit dem Alkohol $R_2OH$ oder einem funktionellen Derivat dieses Alkohols umsetzt,

man zur Erzielung eines Produkts der Formel (I) mit trans-Konfiguration ein Dialkyl-α-cyano-α-$AR_1$-methylphosphonat mit Butyllithium umsetzt, danach das erhaltene Produkt mit tert.-Butyl-1R, trans-2,2-dimethyl-3-formyl-cyclopropan-carboxylat der Formel

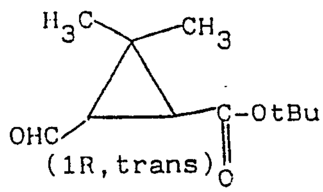

umsetzt, das erhaltene, rohe Säurengemisch der Formel

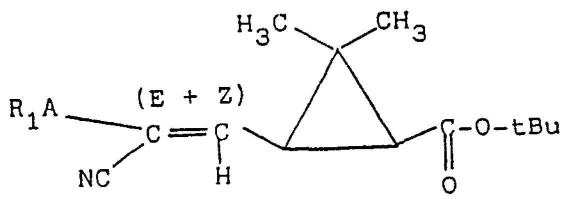

isoliert, hierin gewünschtenfalls die ΔE- und ΔZ-Isomeren trennt, danach gewünschtenfalls die tert.-Butylgruppe entweder mit Trifluoressigsäure bei den ΔZ-Isomeren oder mit p-Toluolsulfonsäure bei den ΔE-Isomeren abspaltet und anschließend gegebenenfalls die so erhaltenen ΔE- und ΔZ-Säuren oder ein funktionelles Derivat dieser Säuren der Einwirkung eines Alkohols $R_2OH$ oder eines funktionellen Derivats dieses Alkohols unterzieht, um die entsprechende Verbindung der Formel (I) zu erhalten.

8. Verwendung der Verbindungen der Formel (I) gemäß einem der Ansprüche 1 bis 5 zur Bekämpfung von Parasiten der Pflanzen und Parasiten von Räumlichkeiten.

9. Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eines der Produkte gemäß einem der Ansprüche 1 bis 5 enthalten.

10. Insektizide Zusammensetzungen, enthaltend als Wirkstoff zumindest eines der Produkte gemäß einem der Ansprüche 1 bis 5.

11. Akarizide Zusammensetzungen, enthaltend als Wirkstoff zumindest eines der Produkte gemäß einem der Ansprüche 1 bis 5.

12. Nematizide Zusammensetzungen, enthaltend als Wirkstoff zumindest eines der Produkte gemäß einem der Ansprüche 1 bis 5.

13. Akarizide Zusammensetzungen für die Bekämpfung von Parasiten warmblütiger Tiere, insbesondere von Zecken und Krätzmilben, dadurch gekennzeichnet, daß sie als Wirkstoff zumindest eines der Produkte gemäß einem der Ansprüche 1 bis 5 enthalten.

14. Zusammensetzungen für die tierische Ernährung, enthaltend als Wirkstoff zumindest eines der Produkte gemäß einem der Ansprüche 1 bis 5.

15. Zusammensetzungen gemäß einem der Ansprüche 9 bis 14, dadurch gekennzeichnet, daß sie außerdem einen Synergisten der Pyrethrinoide, ausgewählt unter Piperonylbutoxid oder Tropital, enthalten.

16. Als neue, industrielle Produkte

2-(Cyano-2-propyloxymethyl)-2-oxo-4,5-dimethyl-1,3,2-dioxaphospholan,

2-(Cyano-ethoxymethyl)-2-oxo-4,5-dimethyl-1,3,2-dioxaphospholan.